(19)

Europäisches
Patentamt
European
Patent Office
Office européen
des brevets

(11)  **EP 3 517 116 A1**

(12)  **EUROPEAN PATENT APPLICATION**
published in accordance with Art. 153(4) EPC

(43) Date of publication:
**31.07.2019  Bulletin 2019/31**

(21) Application number: **17852483.1**

(22) Date of filing: **10.08.2017**

(51) Int Cl.:
*A61K 31/70* [(2006.01)]    *A61K 47/36* [(2006.01)]
*A61K 47/38* [(2006.01)]    *C08J 3/075* [(2006.01)]

(86) International application number:
**PCT/IB2017/054890**

(87) International publication number:
**WO 2018/055463 (29.03.2018 Gazette 2018/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority:  **23.09.2016  MX 2016012389**

(71) Applicants:
• **Sullivan Barrera, Michael O., Jr.**
  **Jalisco, Zapopan 45050 (MX)**

• **Esquivel Solis, Hugo**
  **Jalisco, Tlajomulco De Zuñiga 45645 (MX)**

(72) Inventors:
• **Sullivan Barrera, Michael O., Jr.**
  **Jalisco, Zapopan 45050 (MX)**
• **Esquivel Solis, Hugo**
  **Jalisco, Tlajomulco De Zuñiga 45645 (MX)**

(74) Representative: **Grünecker Patent- und Rechtsanwälte PartG mbB Leopoldstraße 4 80802 München (DE)**

(54)  **A CROSS-LINKED STRUCTURE FOR TISSUE REGENERATION AND ENGINEERING AND THE METHOD FOR SYNTHESISING SAME**

(57)    This invention consists of a reticular structure for tissue regeneration and engineering, and its synthesis process comprising hemicellulose, carboxymethyl cellulose (CMC) and hydroxyethyl cellulose (HEC). The objective of this invention is to create a sustainable reticular structure for tissue regeneration and engineering formed by natural polymers, with a pore size consistent with the natural tissue it intends to imitate; that promotes the growth of tissue and enables the controlling and directing of tissue integration into the structure. A synthesis process of the sustainable reticular structure for tissue regeneration and engineering from natural polymers is also described.

FIG. 8

Printed by Jouve, 75001 PARIS (FR)

EP 3 517 116 A1

## Description

## FIELD OF INVENTION

This invention is related to tissue engineering, particularly to a reticular structure, for the tissue regeneration and engineering, and its synthesis process.

## INVENTION BACKGROUND

Tissue engineering refers to the practice of combining scaffolds, cells and biologically active molecules to create functional tissues; the objective is to collect biotechnological-based knowledge, techniques and methods that enable the design, generation, restoration, maintenance and improvement of damaged tissues or even complete organs. At present, tissue engineering has issues at obtaining a material that possesses a high swelling, hydration, plasticity, flexibility, hardness, in addition to being biocompatible and biodegradable; the material also has to enable the adhesion, growing, differentiation and regeneration of cells, tissues and organs, while also allowing the elaboration of different shapes and sizes for medical use. Another current limiting factor of the existing materials is the lack of control over the creation of a porous reticular structure with a porosity consistent with the natural tissue it intends to imitate.

In general, the materials used in tissue engineering are ceramics, natural or synthetic polymers, and compounds formed of several materials. The most commonly used materials are polymers thanks to their characteristics and versatility. Among the polymer group, hydrogels are materials of great interest since they are of heteropolymeric base with tridimensional networks and an extraordinary capacity to absorb water and different fluids. Hydrogels based on natural polymers, such as those made from proteins and polysaccharides, are widely used in tissue engineering for articular cartilage because, in addition to being biocompatible, they are usually degradable and capable of promoting cell adhesion and proliferation; hydrogels are also immunologically inert.

In the state of art, there are polysaccharide-based hydrogels. For example, the patent application WO2014128354 refers to nanofiber hydrogels made of polysaccharides, which are used in the prevention and control of cicatrization in relation to wound healing or tissue repair.

Another example can be found in the patent application WO2015101711 which describes a modeled membrane comprising polysaccharide nanofibers and compounds to improve wound healing, as well as its use and manufacturing.

Likewise, Tovar-Carrillo Karla L. et al. (2013), in Fibroblast Compatibility on Scaffold Hydrogels Prepared from Agave Tequilana Weber Bagasse for Tissue Regeneration, Ing. Eng. Res., 52 (33), pp 11607-11613, describe a method to make hydrogels from Agave Tequilana Weber Blue Variety cellulose with proved cytocompatibility and biocompatibility properties, obtained by phase inversion of the DMAc-LiCl solution.

Furthermore, hydrogels based on carboxymethyl cellulose (CMC) and hydroxyethyl cellulose (HEC) have been used to obtain thin, transparent films for use in tissue engineering. On the one hand, CMC is a cellulose derivative composed of carboxymethyl groups attached to some hydroxyl groups. The presence of sodium CMC in hydrogels regulates the water absorption capacity and provides hydrogels with increased swelling. On the other hand, HEC is a cellulose derived polymer where ethylene oxide groups have been added to the hydroxyl groups. Overall, HEC promotes the intermolecular reticulation instead of the intramolecular one; this contributes to the stabilization of the chemical network. However, the hydrogels containing CMC, HEC or a combination of both that have been developed to date do not have all the appropriate characteristics for tissue engineering.

One example of hydrogel with CMC can be found in the patent application US2008070997 which describes a method to prepare a gel from alkali metal salt CMC by using an acid or an acid solution. Nevertheless, this method's disadvantage is that, in order to increase the gel's resistance and elasticity, it is necessary to add a water insoluble metal compound or an organic reinforcement to the mix.

Another example is described in the paper by Demitri Christian et al. (2008), Novel superabsorbent cellulose based hydrogels crosslinked with citric acid, Journal of Applied Polymer Science, 110(4), 2453 - 2460; the paper describes a method to elaborate hydrogels with CMC and HEC crosslinked with citric acid, but it has no pore-size control in the final product.

Moreover, in the state of art, there are also hemicellulose-based hydrogels. Hemicellulose is a non-cellulosic heteropolysaccharide consisting of hexoses, pentoses and, in most cases, uronic acids. Hemicellulose has several inherent advantages, including non-toxicity, biocompatibility, biodegradability and suggested effects against cancer (Ying Guan et al., in 2014 [Nanoreinforced hemicellulose-based hydrogels prepared by freeze-thaw treatment]).

In this respect, for its use in a variety of applications, including cell culture, the patent application WO2013112491 refers to a hydrogel composition made of a non-denatured protein, a reticulating agent comprising metallic ions and water, and a polysaccharide selected from cellulose, hemicellulose, xylene, pectin, alginate, chitin and hyaluronic acid.

For its part, the patent application EP2688914 describes a method to make hydrogels from cellulose esters with hemicellulose of crosslinked xyloses, all based on organic waste; however, these hydrogels do not have the appropriate characteristics for tissue engineering.

Ying Guan et al. (2014), in Nanoreinforced hemicellulose-based hydrogels prepared by freeze-thaw treatment, Cellulose, 21(3), 1709-1721, describe the formation of hydrogels from bamboo hemicellulose, polyvinyl alcohol (PVA) and chitin nanotubes using a freezing-thawing method without chemical reticulation. Even though it is said that the invention has three-dimensional networks and good mechanical and thermal properties with possible applications in tissue engineering and medical-biological fields, the non-reticulation of polymers makes the possibly three-dimensional network unstable and reversible to the hydrogel state; therefore, as a porous reticular structure consistent with the tissue it intends to imitate, it is not suitable.

Finally, the patent application WO2015069634 describes essentially spherical microparticles of a polysaccharide reticulated gel comprising one or more linear polysaccharides, with at least one measure unit of 5 to approximately 200 $\mu$m. However, even though this document describes the use of linear polysaccharides forming networks, no porous reticular structure appropriate for tissue engineering is generated in this document.

According to the aforementioned, in the current state of art regarding tissue engineering, there is no material that can provide an appropriate porous reticular structure to serve as a prothesis or tissue filling. Due to the physicochemical behavior, concerning especially link capacity and viscous-elastic behavior, the material need to have the appropriate characteristics of swelling degree, hydration, plasticity, flexibility and hardness; in addition, it must also be biocompatible, biodegradable, and allow the adhesion, growing and differentiation of cells, tissues and organs.

Consequently, efforts have been made to suppress the inconveniences presented by the polymeric hydrogels made from cellulose that are currently used for tissue engineering; this has been addressed by developing a reticular structure for tissue regeneration and engineering that, in addition to resolving the lack of a structure stimulating the growth of tissue (i.e., a porous reticular structure with a porosity consistent with the natural tissue it intends to imitate), enables the controlling and directing of tissue integration into the structure, as well as the capacity to maintain the living cells by allowing vital fluids and nutrients to pass.

## INVENTION OBJECTIVES

Considering the defects of the aforementioned technique, one of the objectives of this is invention is to provide a reticular structure for tissue regeneration and engineering, formed by natural polymers, with a pore size consistent with the natural tissue it intends to imitate.

Furthermore, a purpose of this invention is to deliver a sustainable reticular structure for tissue regeneration and engineering, formed by natural polymers, that stimulates the growth of tissue.

Another objective of this invention is to provide a reticular structure for tissue regeneration and engineering, formed by natural polymers, that enables the controlling and directing of tissue integration into the structure.

An additional purpose of this invention is to deliver a synthesis process of a reticular structure for tissue regeneration and engineering from natural polymers.

This and other objectives can be attained through a reticular structure for tissue regeneration and engineering and a synthesis process in accordance with this invention.

## BRIEF DESCRIPTION OF THE INVENTION

This invention refers to a reticular structure for tissue regeneration and engineering comprising hemicellulose, carboxymethyl cellulose (CMC) and hydroxyethyl cellulose (HEC). Its use allow the cells to enter, migrate and grow throughout the structure. Therefore, the course of cell, tissue and organ regeneration is accelerated; the healing rate is increased; the neogenesis time is reduced, and the resolution is highly improved.

Another aspect of this invention refers to the synthesis process of a reticular structure for tissue regeneration and engineering comprising the following: cold-water solubilization of hemicellulose, carboxymethyl cellulose (CMC) and hydroxyethyl cellulose (HEC) polymers; addition of reticulating agent; heating between 30°C and 120°C from 1 to 8 hours for the reticulating reaction to happen; cooling for the hydrogel to form; repeated freezing and thawing of the hydrogel for the controlled formation of pores; and drying to obtain a reticular structure for tissue regeneration and engineering.

## BRIEF DESCRIPTION OF THE FIGURES

The novel aspects considered to be characteristic traits of this invention will be established focusing on the annexed claims. Nevertheless, some methods, characteristics and several objectives and advantages, will be better understood in the detailed description when read in relation to the annexed figures where:

Figure 1 is a photograph of an example of this invention reticular structure in circular shape according to this invention specific method.

Figure 2 is a photograph of an example of this invention reticular structure in rectangular shape according to this invention specific method.

Figure 3 is a photograph of the porosity ultrastructure and size of the interconnected pores of the reticular structure according to this invention.

Figure 4 is a comparative graph of the swelling percentage (%S) against time in minutes of the reticular structure in accordance with this invention specific method.

Figure 5 is a comparative graph of pulling resistance (MPa) of the reticular structure in accordance with this invention specific method.

Figure 6 is a comparative graph of elongation percentage for traction breaking of the reticular structure in accordance with this invention specific method.

Figure 7 is a comparative graph of cell viability percentage showing the cytocompatibility of the reticular structure in accordance with this invention specific method.

Figure 8 is a microscopic photograph of the reticular structure, in accordance with this invention specific method, completely covered by adhered mammal cells.

Figure 9 is a photograph of the collocation of a collagen commercial implant where skin (in a pig) has been removed.

Figure 10 is a photograph of the collocation of a reticular structure, in accordance with this invention specific method, with a silicone external layer where skin (in a pig) has been removed.

Figure 11 is a photograph of the cicatrization results from the study of biocompatibility and *in vivo* efficacy in a pig using a control which did not used the implant.

Figure 12 is a photograph of the cicatrization results from the study of biocompatibility and *in vivo* efficacy in a pig using a collagen commercial implant.

Figure 13 is a photograph of the results of the cicatrization results from the study of biocompatibility and *in vivo* efficacy in a pig using the reticular structure in accordance with this invention specific method.

Figure 14 is a photograph of a rat's kidney; a third of it had been removed.

Figure 15 is a photograph of the results from a implantability study in *in vivo* mammal organs, 12 weeks after removing one third of the rat's kidney by surgery and implanting the reticular structure in accordance with this invention specific method.

Figure 16 is an enlargement of the area marked as (112) in figure 15.

Figure 17 is a blood urea concentration graph (mg/dL) of the results from the implantability study in *in vivo* mammal organs in healthy rats; i.e. without surgery, and 12 weeks after removing one third of the rat's kidney by surgery (resected) and implanting the reticular structure (resected implanted) in accordance with this invention specific method.

Figure 18 is a blood creatinine concentration graph (mg/dL) of the results from the implantability study in *in vivo* mammal organs in healthy rats; i.e. without surgery, and 12 weeks after removing one third of the rat's kidney by surgery (resected) and implanting the reticular structure (resected implanted) in accordance with this invention specific method.

## DETAILED DESCRIPTION OF THE INVENTION

Surprisingly, it has been found that the reticular structure for tissue regeneration and engineering of this invention, formed of at least hemicellulose, carboxymethyl cellulose (CMC) and hydroxyethyl cellulose (HEC), may have a suitable porosity so the cells from different tissues can enter, migrate and grow throughout the structure; therefore, the course of cell, tissue and organ regeneration is accelerated; the healing rate is increased; and the neogenesis time is reduced.

This invention's reticular structure can be used to induce the *in vitro* and *in vivo* cell growth and differentiation in regenerative medicine, as well as in tissue, organ and prothesis engineering since the structure is biocompatible, biodegradable and with adequate sized pores which allow the adhesion, growing and differentiation of mammal cells, as well as the nutrient flow and the full acceptance of the individuals with implants or bioreactors with adhesive cells.

According to the aforementioned, one of the invention aspects refers to a reticular structure for tissue regeneration and engineering comprising hemicellulose, carboxymethyl cellulose (CMC) and hydroxyethyl cellulose (HEC).

This invention reticular structure is a porous structure with interconnected pores sized between 30 and 500 $\mu$m. Preferably, the size of the interconnected pores of the reticular structure for tissue regeneration and engineering is between 30 a 300 $\mu$m.

As a preferred method for this invention, the reticular structure for tissue regeneration and engineering comprises hemicellulose, CMC and HEC in a 1:5:0.5 to 2:2:1 proportion, respectively, provided that the hemicellulose does not exceed 7% (p/p) with respect to the structure's total weight, and the CMC does not exceed 20% (p/p) with respect to the structure's total weight.

Hemicellulose can have different origins, whether natural or synthetic. In natural occurrence, hemicellulose can be commonly found in the walls of plant cells. For this invention preferred method, hemicellulose is obtained from natural sources, such as plants, wood, plant stems and plant stem cells. In a preferred manner, hemicellulose is obtained from agave plants; and it is even better when it is obtained as a waste resulting from the cellulose obtaining process from agave plants.

Hemicellulose improves the mechanical properties of the reticular structure enabling an increase of tissue regeneration, in addition to bringing more stability to the structure at different temperatures. Agave hemicellulose is preferred since it brings stability at temperatures exceeding those of heat sterilization (130°C).

CMC and HEC are cellulose derivatives. They are obtained through chemical treatment. For this invention preferred method, CMC and HEC are obtained through chemical treatment in cellulose alkaline medium; cellulose can be found in cell walls of plants. In a preferred manner, CMC and HEC are obtained from agave plant cellulose. Agave CMC and HEC are preferred for the formation of the reticular structure due to their suitable characteristics for tissue regeneration and engineering and to take advantage of the agave bagasse waste in the tequila and mezcal industry.

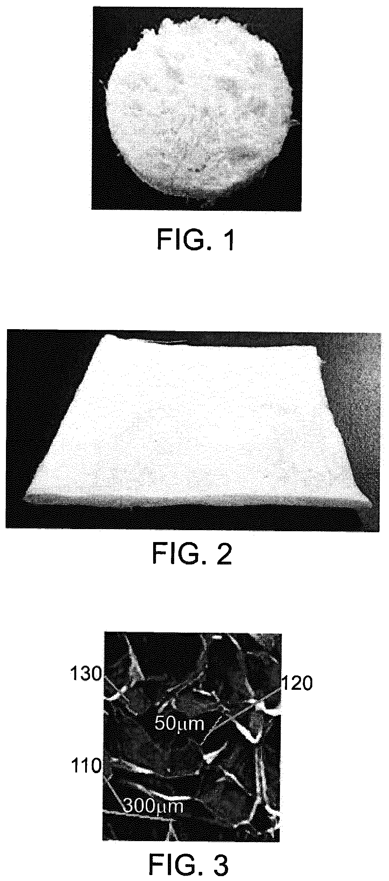

It is important to mention that CMC modulates the water absorption capacity and provides the reticular structure with electrostatic charges attached to the network, which has a double effect on the swelling capacity. On the one hand, the electrostatic repulsion established by same sign charges forces the polymer chains into a more elongated state when compared to that of a neutral network; this increases the swelling and controls the pore size. On the other hand, ions of opposite charges in the gel guarantee the macroscopic electric neutrality instead and induce the entrance of more water. Moreover, HEC promotes the intermolecular reticulation instead of the intramolecular one; this contributes to the stabilization of the reticular structure. This invention reticular structure can be obtained in different sizes and shapes that go from 0.3 cm x 0.5 cm films to 10 cm x 100 cm films with lengths ranging from 0.5 cm to several meters. Some examples can be seen in figures 1 and 2. An example of a circular reticular structure (100) is shown in figure 1, and a rectangular structure (101) can be seen in figure 2.

Additionally, three-dimensional structures can be obtained, such as cylinders, cones, spheres, triangular prisms, square prisms, rectangular prisms, pentagonal prisms, hexagonal prisms and pyramids, as well as three-dimensional irregular shapes, i.e. any three-dimensional shape made from a mold, cut or milling. Sizes can go from 0.3 cm$^3$ to 15 cm$^3$.

Shapes and sizes adapt to the structure that will be implanted in a tissue or organ. For example, a dermal substitute is obtained as a net or membrane of 0.3 cm x 15 to 20 cm; cubes for bone filling can be from 0.5 cm$^3$ to 2-4 cm$^3$; granules for dental pulp filling can be 0.2 cm$^3$ to 0.3 cm$^3$; sheets for subcutaneous filling can have a width of 0.2 cm with different lengths and thickness in cm. Any shape and size can be obtained depending on the application.

This invention reticular structure has different tensile strength values (flexibility, hardness and stiffness) which are optimal for medical use. These values are necessary to reflect the true nature and biomechanical characteristics of the anatomical parts of the tissue and organs that are going to be replaced in the body; structural properties are represented by a relation between force, deformation, stress and tension. The values found in this reticular structure, in accordance with this invention, exceed the values of 21.6 MPa in human skin obtained by Aisling Ní Annaidh et al. (2012, Characterising the Anisotropic Mechanical Properties of Excised Human. Journal of the Mechanical Behavior of Biomedical Materials. 5: 139-148) and of 27.2 MPa obtained by Gallagher AJ et al. (Dynamic Tensile Properties of Human Skin http://researchrepository.ucd.ie/handle/10197/4772); even with higher tear resistance in soft organs like the stomach (<20 MPa), liver (<15 MPa), heart (<12 MPa) and lung (<25 MPa), studied by Saraf H. et al. (2007, Mechanical properties of soft human tissue under dynamic loading. Journal of Biomechanics 40:1960-1967); and values similar to those of articular cartilage (40 MPa) obtained by Triona McCormack and Joseph M. Mansour (1998, Reduction in tensile strength of cartilage precedes surface damage under repeated compressive loading in vitro. Journal of Biomechanics 31:55-61).

Another aspect of this invention refers to the synthesis process of a reticular structure for tissue regeneration and engineering comprising the following: cold-water solubilization of hemicellulose, carboxymethyl cellulose (CMC) and hydroxyethyl cellulose (HEC) polymers; addition of reticulating agent; heating between 30°C and 120°C from 1 to 8 hours for the reticulating reaction to happen; cooling for the hydrogel to form; repeated freezing and thawing of the hydrogel for the controlled formation of pores; and drying to obtain a reticular structure for tissue regeneration and engineering.

As a preferred method for this invention, the stage of cold-water solubilization of hemicellulose, carboxymethyl cellulose (CMC) and hydroxyethyl cellulose (HEC) polymers was carried out between 0 and 8°C while vigorously stirring in water until they completely dissolve. Cold temperature helps stabilizing the hydrogen bonds between the different sugar chains; this enables the desired porous structure in order to obtain interconnected pores sized between 30 and 500 $\mu$m, preferably between 30 and 300 $\mu$m. As a preferred method for this invention, the reticulating agent is added to the mixture of hemicellulose, CMC and HEC in a proportion of 0.5 to 20% (p/p) with respect to the mixture.

For this invention, a reticulating agent refers to any chemical compound or a chemical compound mixture that enables the formation of chemical bonds between molecular chains in order to create a three-dimensional net. As a preferred method for this invention, the reticulating agent is a covalent, non-toxic and biocompatible reticulating agent selected preferably from among carboxylic acids, compounds with carbonyl groups, or any of their salts. Specifically, the reticulating agent is citric acid, a tricarboxylic acid that at temperatures between 0 and 30°C behaves as a triacid, and at temperatures above 100°C behaves as a diacid.

As a preferred method for this invention, in the stage of repeatedly freezing-thawing the hydrogel for the controlled formation of pores, the hydrogel obtained during the freezing stage is frozen at temperatures between -20 and -80°C for 8 to 24 hours; subsequently, the hydrogel is thawed for 1 to 3 hours at room temperature. This freezing-thawing cycle is repeated at least once.

By repeating this freezing-thawing stage, the pore formation can be optimized to get the desired size, and the structure is stabilized due to the biomaterial's resilience.

The hemicellulose, CMC and HEC polymers have several hydrophilic groups, such as hydroxyl groups, which creates a strong interaction with water. The thermal properties of these polymers and water are heavily influenced by this interaction. Thus, the water strongly associated with the polymer's matrix is generally called non-freezing water, while the less strongly associated fractions of water show a significant overcooling fusion/crystallization point, and a significantly lower enthalpy when compared to that of common water. These fractions of water less strongly associated with the polymer's matrix are called freezing water. During each freezing cycle, water crystals with different sizes are formed- The less strongly associated water attached to a polymer will form these crystals first, while the strongly associated water will do it later. Therefore, in each freezing-thawing cycle, the hydrogel density will increase due to the displacement of the fractions of water that froze first (crystals). Thus, as the freezing-thawing cycles are performed, a swelling/densification balance is attained; the hydrogel's strength and hardness are increased; smaller crystals are formed and, as a result, smaller pores. Hence, the number of freezing-thawing cycles will depend on the desired pore size and how hard the structure is going to be. It has been observed that, after 5 to 6 cycles, a balance is attained; i.e., there are no more modifications on the mechanical properties or the pore size. In this manner, in a specific method for this invention, 2 freezing-thawing cycles are enough for the reticular structure of this invention to be suitable as a dermal substitute or as implant in soft organs. In another specific method for this invention, in accordance with the invention principles, 4 to 5 freezing-thawing cycles are needed for the obtained reticular structure to be suitable as a substitute or implant of bone tissue.

As a preferred method for this invention, during the drying stage to obtain a reticular structure for tissue regeneration and engineering, the frozen hydrogel dehydrates through cold drying (sublimation or lyophilization) or through phase changes with acetone and leaving it to evaporate in a vacuum chamber at room temperature. All of this results in a reticular structure in accordance with this invention.

This invention will be better understood with the following examples, which are for illustrative purposes only to allow a full understanding of the preferred methods for this invention; this does not mean that there are no other methods that can be put into practice based on the aforementioned detailed description.

## EXAMPLES

These examples will use the following mixtures abbreviations:

CMC-HH: carboxymethyl cellulose-hydroxyethyl cellulose-hemicellulose
CMC-HE: carboxymethyl cellulose-hydroxyethyl cellulose
CMC: carboxymethyl cellulose

## Example 1. Obtaining agave hemicellulose

For illustrative purposes, here it is explained how it is possible to obtain hemicellulose from agave plant to use it in the preparation of a reticular structure in accordance with these invention principles.

Agave hemicellulose is obtained by preconditioning agave fibers; the process consists in washing, cut and removal of waxes. After washing the fibers 3 to 5 times with distilled water at a 10:1 vol/wt proportion for approximately 10 minutes each in order to eliminate sugar residues. the fibers are left to drip and dry in a vacuum furnace at approximately 50°C. Approximately 10 g to 100 g of dried agave fibers are then submerged in 1000 mL of 10 to 20% NaOH solution at 100 °C for around 5 to 12 hours; they can also be submerged in 0.1 to 0.6 M NaOH solution at 45°C for around 1 to 3 hours until a black liquor is obtained. Hemicellulose and cellulose fibers are obtained separately through filtration, and the lignin is discarded. The hemicellulose and cellulose fibers are then processed in the same way but separately and simultaneously. The fibers are washed several times with distilled water at a 5 to 10:1 proportion to eliminate NaOH residues; afterwards, they are submerged in a 0.1 M NaOH solution at 45°C for around 3 hours. Consecutively, fibers receive successive treatments with 0.5%, 1%, 2% and 3% $H_2O_2$ for 3 hours each at 45°C, with a pH of 11.5. Fibers are then washed submerging them in 1000 mL of distilled water at a 5:1 vol/wt proportion to eliminate the $H_2O_2$ residues at room temperature until a pH 7 is obtained.

Finally, fibers are left to drip and dry at room temperature with vacuum pressure. This is when pure hemicellulose fibers have been obtained for the processes of this invention's reticular structure. In addition, cellulose fibers that were processed separately are also obtained.

## Example 2. Obtaining agave carboxymethyl cellulose

For illustrative purposes, here it is explained how it is possible to obtain CMC from agave plant to use it in the preparation of a reticular structure in accordance with these invention principles.

For the agave CMC synthesis process, the obtained cellulose fibers mentioned above are added to 1000 mL of a 4% $H_2SO_4$ solution and stirred during 2 hours at 100°C. The fibers are then washed 5 times with distilled water at a 10:1 vol/wt proportion for 10 minutes each time to eliminate the $H_2SO_4$ residues. Fibers are then left to drip and dry in a vacuum furnace at 50°C for 3 days. The stirring is done to homogenize the suspension. Consecutively, 50 mL of NaOH are added while vigorously stirring for 30 minutes at room temperature. Afterwards, 18 g of monochloroacetic acid are slowly added during a 30-minute period. Then, the circulation of hot water is started inside the jacketed reactor for the reaction to occur at a temperature of 55 to 70°C during a mean time of 3.5 to 5 hours. After this time, the mixture is cooled down and filtrated. The obtained fibrous material is mixed in the same reactor with 300 mL of 70% v/v methanol and neutralized with 90% acetic acid. Fibers are then separated again by filtration and mixed with 300 mL of 70% v/v ethanol while stirring for 10 minutes. The mixture is left for 10 minute to facilitate the separation. The filtration and stirring with ethanol is repeated at least 1 time. Finally, the product is washed with absolute methanol and dried in an oven at 60°C

### Example 3. Obtaining agave hydroxyethyl cellulose (HEC)

For illustrative purposes, here it is explained how it is possible to obtain HEC from agave plant to use it in the preparation of a reticular structure in accordance with these invention principles.

For the agave hydroxyethyl cellulose synthesis, a 1:6 urea:NaOH solution is gradually cooled down in distilled water at 12°C; then 5 to 10 g of the dry agave cellulose fibers obtained in example 1 are immediately added while vigorously stirring for 1 hour at room temperature. To obtain the transparent cellulose additive, the formed alkaline cellulose is filtered until the sodium hydroxide and cellulose solution proportion is 1:3 in weight. 5 mL of 2-chloroethanol are slowly added to the aforementioned cellulose solution. The mixture is stirred at approximately 25°C for 1 hour; the temperature is then increased to 50°C for 8 hours. The obtained reaction product is neutralized with acetic acid and dyalized with water. Finally, the solution is lyophilized.

### Example 4. Obtaining a reticular structure in accordance with this invention

For illustrative purposes, here it is explained how it is possible to obtain a reticular structure in accordance with these invention principles from agave hemicellulose, CMC and HEC obtained in the aforementioned examples.

A 100-mL aqueous solution is prepared with 2 g of agave hemicellulose, 6 g of agave CMC, 3 g of agave HEC, and vigorously cold stirring (min 100 rpm) at 4°C. The CMC dissolution is slow; therefore, CMC should be added at the end. Once a clear solution with a slight viscosity increase is obtained, 5 g of citric acid are added as reticulating agent. The mixture is heated to 60°C, and is kept like that for 6 hours. After the mixture has cooled down at room temperature, it is transferred to molds to form the hydrogel. Afterwards, the mixture is frozen at -20°C and kept like that for 8 hours. After that time, the hydrogel is thawed for 3 hours at room temperature. This freezing-thawing cycle is repeated twice. Finally, the hydrogel is frozen again at -40°C and dehydrated through lyophilization for 24 to 48 hours in order to obtain a reticular structure with a mean pore size of 200 μm. The lyophilization uses fast cooling to produce thermodynamic instability inside a system, causing phase separation. The water is then eliminated by sublimation (ice to vapor) at high vacuum (0.133 mBar); the process leaves holes in the regions where water had previously been.

### Example 5. Obtaining a reticular structure in accordance with this invention using a proportion of hemicellulose, CMC and HEC different from those in example 4.

For illustrative purposes, a second test was performed to obtain a reticular structure in accordance with these invention principles from agave hemicellulose, CMC and HEC obtained in previous examples.

A 100-mL aqueous solution is prepared with 0.5 of agave hemicellulose, 3 g of agave CMC and 1 g of agave HEC, and vigorously cold stirring (min 100 rpm) at 4°C. Then, 3 g of citric acid are added as reticulating agent. The mixture is heated at 60°C and kept like that for 6 hours. After the mixture has cooled down at room temperature, it is transferred to molds to form the hydrogel. Afterwards, the mixture is frozen at -20°C and kept like that for 8 hours. After that time, the hydrogel is thawed for 3 hours at room temperature. This freezing-thawing cycle is repeated 4 times. Finally, the hydrogel is frozen again at -40°C and dehydrated through phase change with acetone for 24 hours, and leaving it to evaporate in a vacuum chamber at room temperature for 48 hours.

Figure 3 shows a photograph of the porosity ultrastructure and size of the interconnected pores in the reticular structure obtained in this example. It shows interconnected pores, a pore with a 300- μm diameter (100), a pore with a 50- μm diameter, and a wall of the reticular structure (130).

### Example 6. Swelling and hydration test

For illustrative purposes, a test was performed to determine the swelling and hydration percentages of the reticular structure obtained in the previous example in accordance with these invention principles.

The reticular structure prepared in example 4 (CMC-HH) was compared to a CMC-HEC-based reticular structure and to a CMC-based reticular structure.

First, the reticular structures were counted in 0.5 x 0.5 cm samples, and the initial weight was recorded (Md). Then, each sample was transferred to separate flasks, and 5 mL of DMEM medium were added with a 7.4 pH at 37°C; the flasks then were hermetically sealed. The flasks were introduced in an oven at 37°C and taken out every 2 minutes during the first 10 minutes to weigh them. This procedure was repeated every 10 minutes until the sample attained a constant weight. At least three samples were analyzed to prove repeatability. The superficial water in each reticular structure was taken with filter paper. The swollen reticular structure were weighed and the water absorption percentage was calculated with the following equation:

$$S\% = \frac{Ms - Md}{Md} X\ 100$$

Where S is the water absorption capacity at equilibrium (%)

Ms is the weight of the swollen reticular structure (g)

Md is the weight of the dry reticular structure (g)

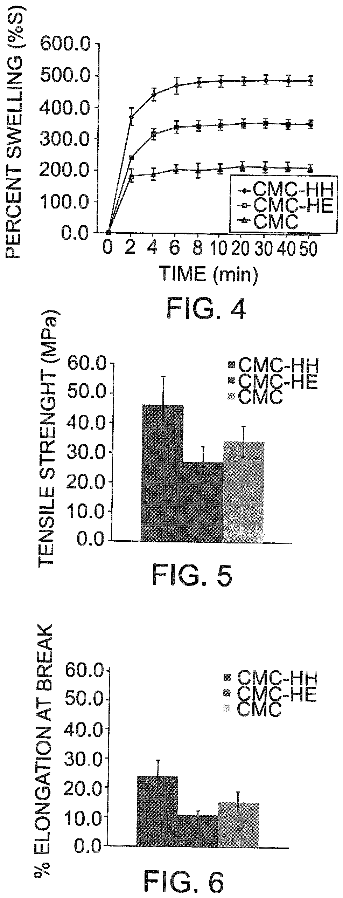

Figure 4 shows a swelling percentage comparative graph (S%) against time in minutes of this invention reticular structure (CMC-HH); of a CMC-HEC-based reticular structure (CMC-HE); and of a CMC-based reticular structure (CMC). An improved behavior of the reticular structure can be seen in accordance with this invention.

## Example 7. Tensile strength (flexibility, hardness and stiffness)

A test was performed to determine the tensile strength of the reticular structure obtained in example 4 in accordance with these invention principles.

The reticular structure prepared in example 4 (CMC-HH) was compared to a CMC-HEC-based reticular structure and to a CMC-based reticular structure.

The tensile strength values of the reticular structures were measured with Hounsfield H10KS tensile strength meter: 50 mm/min, temperature 25°C, and relative humidity 60%. At least three samples were analyzed to prove repeatability. Figure 5 shows a tensile strength comparative graph (MPa) of this invention reticular structure (CMC-HH); of a CMC-HEC-based reticular structure (CMC-HE); and of a CMC-based reticular structure (CMC). Figure 6 shows a comparative graph of elongation percentage for traction breaking of this invention reticular structure (CMC-HH); of a CMC-HEC-based reticular structure (CMC-HE); and of a CMC-based reticular structure (CMC). Both graphs show a better performance for the reticular structures in accordance with this invention. These values reflect the biomechanical characteristics of the anatomical parts of the tissue and organs that are going to be replaced; the values obtained for this invention reticular structure are suitable for its use in different organs and tissues.

## Example 8. *In vitro* biocompatibility with human fibroblasts (HFF-1)

A test was performed to determine the *in vitro* biocompatibility with human fibroblasts (HFF-1) of the reticular structure obtained in example 4 in accordance with these invention principles.

The reticular structure prepared in example 4 (CMC-HH) was compared to a CMC-HEC-based reticular structure and to a CMC-based reticular structure.

The biocompatibility of the reticular structures was measured *in vitro* through the viability of human skin cells (fibroblasts; HFF1 ATCC SCRC-1041) cultured on the materials. The tetrazolium salts (MTT) reduced to formazan crystals (violet and insoluble) were used to determine only the percentage of living cells since dead cells do not reduce MTT. As a 100% reference, a cell culture (HFF1) in the same conditions but without the reticular structures was used. With a paper punch, circles were cut from the reticular structures; they were sterilized with hydrogen peroxide plasma and placed in cell culture trays with 96 wells, one per well. Afterwards, 75,000 fibroblasts were cultured in a DMEM pH 7.4 medium and incubated at 37°C for 7 days. After 7 days of incubation, the wells were washed twice with PBS and an MTT solution was added and left for a 24-hour incubation. After this time, cells were fixed with 70% ethanol, and the formazan crystals were dissolved with isopropanol. A color measurement at 550 nm and 690 nm was performed to subtract the background reading. The sample values are shown in percentages of the control sample absorbance (100% of viable cells). Percentages over 90% are considered biocompatible.

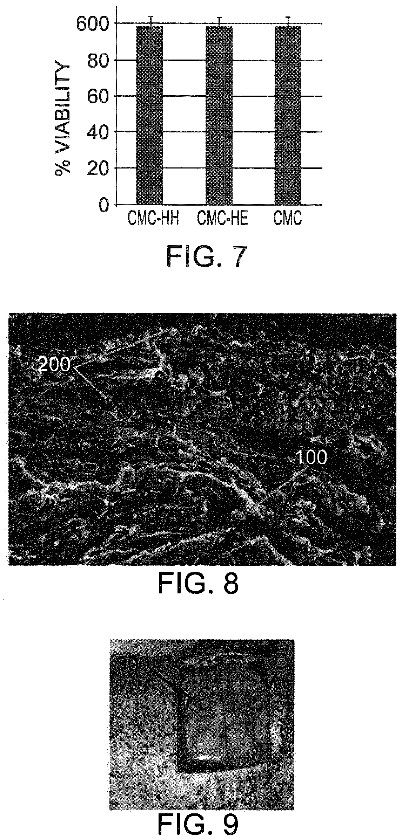

The cell viability percentages showing cytocompatibility of the used reticular structure are shown in figure 7, where this invention reticular structure is CMC-HH; the CMC-HEC-based reticular structure is CMC-HE; and the CMC-based reticular structure is CMC. All the analyzed reticular structures are biocompatible, but the reticular structured prepared in example 4 (CMC-HH), in accordance with this invention, has the highest viability percentage.

Figure 8 shows a microscopic photograph of the reticular structure prepared in example 4 in accordance with these invention principles; the photograph was taken during the epithelialization process with human skin fibroblast (HFF-1). A reticular structure is observed, completely covered by these cells that adhered, differentiated and produced extracellular matrix. In this figure 8, the reticular structure (100) is shown as a fibrous material with a lighter tone, and the human skin cells (200) that covered the structure are shown as semispherical or spherical elements with a darker tone. The darkest parts of the photograph correspond to the pores of the reticular structure (100).

## Example 9. *In vivo* biocompatibility and efficacy in pigs

A test was performed to determine the *in vivo* biocompatibility and efficacy of the reticular structure obtained in example 4 in accordance with these invention principles.

To perform the study, six pigs were used, and each pig was considered as an experimental unit. Three incision of 8 cm x 8 cm were done in each pig, going down the vertebral column with 4 cm between each ipsilateral incision and 4 cm from the animal's neck. The skin and subcutaneous tissue were removed at the adipose panicle level. The three treatments for each incision in each pig were the following:

1) Collagen commercial implant (Integra® Dermal Regeneration Template, obtained in 2016), which comprises a silicone external layer and an internal layer made up of collagen obtained from cows and glycosaminoglycan obtained from shark cartilage

2) Secondary intention healing (no treatment)

3) Reticular structure obtained in example 4 in accordance with these invention principles with a silicone external layer

The treatment site was alternated in each pig to avoid implant site bias. Each treatment site was covered with a porous, transparent sheet (Mepitel® Mölnlycke Healthcare AB, obtained in 2016) having adherent properties to reduce the trauma caused by the dressing changes; a nanocrystalline silver dressing (Acticoat® Smith & Nephew Ltd, obtained in 2016) to treat infected wounds and minimize the infection; a polyurethane film covered with polyacrylate (Hypafix®, obtained in 2016) to cover the edges; and a dressing to keep everything in place during the study period.

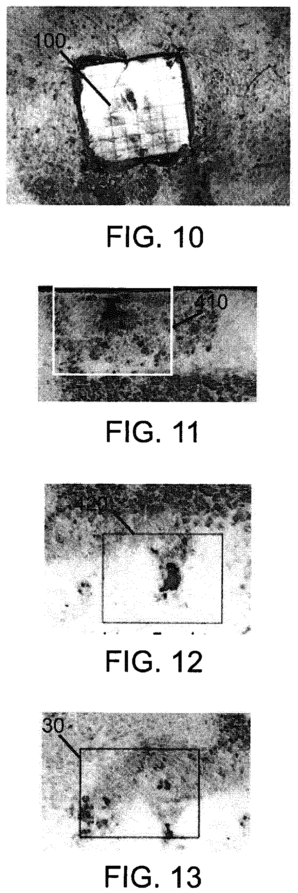

Figure 9 shows the implantation of the commercial implant (300) where the skin of the pig was removed (the pig's skin is yellow due to the antiseptic). Figure 10 shows the reticular structure obtained in example 4, (100) in accordance with these invention principles, being implanted where the pig's skin was removed.

A clinical review was performed to check the wound's evolution (high definition and thermographic photographs), food consumption, and a graft biopsy was carried out at the end of the experimentation.

The study results can be observed in figures 11, 12 and 13. Figure 11, the square (410), shows the secondary intention (natural) healing result without an implant. Figure 12, square (20), shows the healing result using a collagen commercial implant. Figure 13, square (430), shows the primary intention healing result using this reticular structure in accordance with these invention principles. In the three cases, there were no adverse effects associated with the implants, and the physiological parameters were not altered. The pigs' clinical performance was confirmed by the absence of abnormalities, based on the pigs' food and water consumption and their body weight.

The macro and micro analysis showed a well-established regeneration in the case of both treatment sites with implants when compared to the control without treatment. However, the reticular structure obtained in example 4, in accordance with these invention principles, showed a better performance for tissue regeneration than that of the commercial reticular structure in terms of final recovery (4/6 vs 2/6 animals). Only in the case of this reticular structure, in accordance with these invention principles (figure 13), a complete integration can be observed along with a complete closing of the wound and a full regeneration of the skin (thickness, color and hair).

**Example 10. Implantation study in rat kidney**

A test was performed to determine the *in vivo* biocompatibility and efficacy of the reticular structure obtained in example 4 when used as a kidney implant in rats in accordance with these invention principles.

A third of 4-week-old Wistar rats' kidneys was dried, and this invention reticular structure was implanted in the kidney's place with same shape as the extirpated tissue. The animals were kept under controlled conditions for 6 weeks, and the urea and creatinine production was measured throughout the study. The implant impression in the tissue was asses through histological and macroscopic media. F

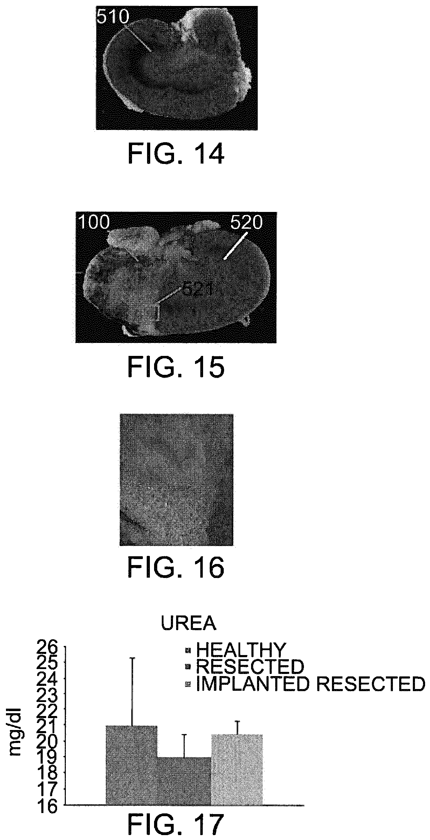

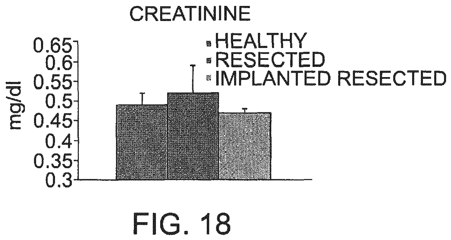

Figure 14 shows a rat kidney without implant (510) that was used as a no-regeneration control for the implantability study in *in vivo* mammal organs, 12 weeks after the kidney was removed without regeneration. Figure 15 shows a rat kidney (520) with this invention reticular structure obtained in example 4. Figure 16 shows an enlargement of the (521) section of the kidney (520) in figure 15; a complete integration is observed along with the vascularization and irrigation of this invention reticular structure without any abnormalities in the receiving tissue. Additionally, figures 17 and 18 show urea and creatinine blood concentration graphs at the end of the study in healthy rats, rats without a third of their kidney, and rats with this invention reticular structure obtained in example 4 as an implant. According to the results, this invention reticular structure was well tolerated and implanted without altering the normal kidney functions.

According to the aforementioned, for a subject matter technician, it will be evident that the reticular structure methods for tissue regeneration and engineering, as well as its synthesis process described above, have only illustrative purposes since a subject technician can perform several variations of the method; for example mixtures with other polymers or molecules used in tissue regeneration and engineering. Consequently, this invention includes all the methods that a subject matter technician could formulate based on the concepts presented in this description.

**Claims**

1. The reticular structure for tissue regeneration and engineering is composed of hemicellulose, carboxymethyl cellulose (CMC) and hydroxyethyl cellulose (HEC).

2. The reticular structure for tissue regeneration and engineering, in accordance with claim 1, additionally comprises a porous structure with interconnected pores sized between 30 and 500 $\mu$m.

3. The reticular structure for tissue regeneration and engineering in accordance with claim 2, has interconnected pores sized between 30 and 300 $\mu$m.

4. The reticular structure for tissue regeneration and engineering, in accordance with claim 1, comprises hemicellulose, CMC and HEC in a 1:5:0.5 to 2:2:1 proportion, respectively.

5. The reticular structure for tissue regeneration and engineering, in accordance with claim 4, is also **characterized by** the hemicellulose which cannot exceed 7% (p/p) with respect to the total weight of the reticular structure.

6. The reticular structure for tissue regeneration and engineering, in accordance with claim 4, is **characterized by** CMC which cannot exceed 20% (p/p) with respect to the total weight of the reticular structure.

7. The reticular structure for tissue regeneration and engineering, in accordance with claim 1, obtains the hemicellulose, CMC and HEC from agave plants.

8. The reticular structure for tissue regeneration and engineering, in accordance with claim 1, can be obtained in different shapes and sizes.

9. The reticular structure for tissue regeneration and engineering, in accordance with claim 8, has different shapes and sizes that can adapt to the structure of the tissue or organ.

10. The reticular structure for tissue regeneration and engineering, in accordance with claim 1, has higher tensile strength values when compared to the human skin values of 21.6 MPa.

11. The synthesis process of a reticular structure for tissue regeneration and engineering comprises the following: a) cold-water solubilization of hemicellulose, carboxymethyl cellulose (CMC) and hydroxyethyl cellulose (HEC) polymers; b) addition of reticulating agent; c) heating between 30°C and 120°C from 1 to 8 hours for the reticulating reaction to happen; d) cooling for the hydrogel to form; e) repeated freezing and thawing of the hydrogel for the controlled formation of pores; and f) drying to obtain a reticular structure for tissue regeneration and engineering.

12. The synthesis process of a reticular structure for tissue regeneration and engineering, in accordance with claim 11, is **characterized by** the cold-water solubilization of hemicellulose, CMC and HEC polymers. The process is carried out at 0 to 8°C while vigorously stirring in water until the elements are completely dissolved.

13. The synthesis process of a reticular structure for tissue regeneration and engineering, in accordance with claim 11, is **characterized by** the reticulating agent which is added in 0.5 to 20% (p/p) in relation to the mixture.

14. The synthesis process of a reticular structure for tissue regeneration and engineering, in accordance with claim 11, is **characterized by** the covalent, non-toxic, biocompatible reticulating agent.

15. The synthesis process of a reticular structure for tissue regeneration and engineering, in accordance with claim 14, is **characterized by** the reticulating agent which is selected preferably from among carboxylic acids, compounds with carbonyl groups, or any of their salts

16. The synthesis process of a reticular structure for tissue regeneration and engineering, in accordance with claim 15, is **characterized by** the reticulating agent which is citric acid.

17. The synthesis process of a reticular structure for tissue regeneration and engineering, in accordance with claim 11, is **characterized by** the cooling stage of the mixture at room temperature in order to form the hydrogel.

18. The synthesis process of a reticular structure for tissue regeneration and engineering, in accordance with claim 17, is **characterized by** the mixture being at room temperature and poured into molds of suitable sizes consistent with the tissue it intends to regenerate or mimic.

19. The synthesis process of a reticular structure for tissue regeneration and engineering, in accordance with claim 11, is **characterized by** the freezing-thawing stage where the hydrogel is frozen at -20 to -80°C for 8 to 24 hours; the hydrogel is later thawed for 1 to 3 hours at room temperature.

20. The synthesis process of a reticular structure for tissue regeneration and engineering, in accordance with claim 11, is **characterized by** the hydrogel freezing-thawing stage which is repeated at least once.

21. The synthesis process of a reticular structure for tissue regeneration and engineering, in accordance with claim 20, is **characterized by** the hydrogel freezing-thawing stage which is repeated twice in order to obtain a reticular structure suitable as dermal substitute or as soft organ implant.

22. The synthesis process of a reticular structure for tissue regeneration and engineering, in accordance with claim 20, is **characterized by** the hydrogel freezing-thawing stage which is repeated four to five times in order to obtain a reticular structure suitable as substitute or implant of bone tissue.

23. The synthesis process of a reticular structure for tissue regeneration and engineering, in accordance with claim 11, is **characterized by** the drying stage where the frozen hydrogel is dehydrated through cold drying.

24. The synthesis process of a reticular structure for tissue regeneration and engineering, in accordance with claim 11, is **characterized by** the drying stage where the frozen hydrogel is dehydrated through phase change with acetone, leaving it to evaporate in a vacuum chamber at room temperature.

25. A reticular structure comprising hemicellulose, carboxymethyl cellulose (CMC) and hydroxyethyl cellulose (HEC) for tissue regeneration and engineering was used.

26. A reticular structure comprising hemicellulose, carboxymethyl cellulose (CMC) and hydroxyethyl cellulose (HEC) for tissue regeneration and engineering was used as a substitute or implant of tissue or organs.

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

FIG. 14

FIG. 15

FIG. 16

FIG. 17

CREATININE

**FIG. 18**

# INTERNATIONAL SEARCH REPORT

| International application No. |
| --- |
| PCT/IB2017/054890 |

**A.    CLASSIFICATION OF SUBJECT MATTER**

(CIP) A61K31/70, 47/36, 47/38, C08J3/075   (2017.01)

According to International Patent Classification (IPC) or to both national classification and IPC

**B.    FIELDS SEARCHED**

Minimum documentation searched (classification system followed by classification symbols)

(CIP) A61K31/70, 47/36, 47/38, C08J3/075 (CPC) A61K31/715, 31/716, 47/36, 47/38

Documentation searched other than minimum documentation to the extent that such documents are included in the fields searched

Electronic data base consulted during the international search (name of data base and, where practicable, search terms used)

STN, Esp@cenet, USPTO, Derwent, Google,  Dialog, Epoque, INAPI

**C.    DOCUMENTS CONSIDERED TO BE RELEVANT**

| Category* | Citation of document, with indication, where appropriate, of the relevant passages | Relevant to claim No. |
| --- | --- | --- |
| X | US2007053960 (A1)  (BOARD OF REGENTS, THE UNIVERSITY OF TEXAS SYSTEM) 08/03/2007 <br> **The whole document** | 1, 8, 9, 10, 25 y 26 |
| T | CHEN, H. Biotechnology of Lignocellulose, Theory and Practice. Chapter 2, Chemical composition and structure of natural lignocellulose, (2014), XVIII:510 ISBN:978-94-007-6897-0 DOI 10.1007/978-94-007-6898-7 (Ver Sección 2.4.1, página 43) | |
| T | LIU, J. et al. Hemicellulose-reinforced nanocellulose hydrogels for wound healing application. Cellulose (2016 oct), 23,5:3129-3143 DOI 10.1007/s10570-016-1038-3 **The whole document** | |

☒ Further documents are listed in the continuation of Box C.        ☒ See patent family annex.

| | |
| --- | --- |
| *    Special categories of cited documents: <br> "A"  document defining the general state of the art which is not considered to be of particular relevance <br> "E"  earlier application or patent but published on or after the international filing date <br> "L"  document which may throw doubts on priority claim(s) or which is cited to establish the publication date of another citation or other special reason (as specified) <br> "O"  document referring to an oral disclosure, use, exhibition or other means <br> "P"  document published prior to the international filing date but later than the priority date claimed | "T"  later document published after the international filing date or priority date and not in conflict with the application but cited to understand the principle or theory underlying the invention <br> "X"  document of particular relevance; the claimed invention cannot be considered novel or cannot be considered to involve an inventive step when the document is taken alone <br> "Y"  document of particular relevance; the claimed invention cannot be considered to involve an inventive step when the document is combined with one or more other such documents, such combination being obvious to a person skilled in the art <br> "&"  document member of the same patent family |

| Date of the actual completion of the international search | Date of mailing of the international search report |
| --- | --- |
| 31/10/2017        31 October 2017 | 27/11/2017        27 November 2017 |

| Name and mailing address of the ISA/ <br> INAPI, Av. Libertador Bernardo O'Higgins 194, Piso 17, Santiago, Chile | Authorized officer |
| --- | --- |
| Facsimile No. | Telephone No. |

Form PCT/ISA/210 (second sheet) (January 2015)

## INTERNATIONAL SEARCH REPORT

International application No.

PCT/IB2017/054890

C (Continuation).    DOCUMENTS CONSIDERED TO BE RELEVANT

| Category* | Citation of document, with indication, where appropriate, of the relevant passages | Relevant to claim No. |
|---|---|---|
| A | XIANG-MING QI, et al. Preparation and characterization of blended films from quaternized hemicelluloses and carboxymethil cellulose. Materials 2016, 9,4:2-12 DOI: 10.3390/ma9010004 *The whole document* | |
| A | LIU, Y et al. Superabsorbent sponge and membrane prepared by polyelectrolyte complexation of carboxymethyl cellulose/hydroxyethyl cellulose-Al3+. (2015) [En Línea] BioResources.com 10(4):6479-6495 [Recuperado 31/10/2017] Recuperado de: <http://ojs.cnr.ncsu.edu/index.php/BioRes/article/view/BioRes_10_4_6479_Liu_Superabsorbent_Sponge_Membrane_Polyelectrolyte/3776> **The whole document** | |

Form PCT/ISA/210 (continuation of second sheet) (January 2015)

INTERNATIONAL SEARCH REPORT
Information on patent family members

| International application No. |
| :--- |
| PCT/IB2017/054890 |

| Patent document cited in the search report | Publication date | Patent family member(s) | Publication date |
| :---: | :---: | :---: | :---: |
| US2007053960 A1 | 08-03-2007 | US8951551 B2 | 10-02-2015 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2014128354 A
- WO 2015101711 A
- US 2008070997 A
- WO 2013112491 A
- EP 2688914 A
- WO 2015069634 A

### Non-patent literature cited in the description

- **TOVAR-CARRILLO KARLA L. et al.** Fibroblast Compatibility on Scaffold Hydrogels Prepared from Agave Tequilana Weber Bagasse for Tissue Regeneration. *Ing. Eng. Res.,* 2013, vol. 52 (33), 11607-11613
- **DEMITRI CHRISTIAN et al.** Novel superabsorbent cellulose based hydrogels crosslinked with citric acid. *Journal of Applied Polymer Science,* 2008, vol. 110 (4), 2453-2460
- **YING GUAN et al.** *Nanoreinforced hemicellulose-based hydrogels prepared by freeze-thaw treatment,* 2014
- **YING GUAN et al.** Nanoreinforced hemicellulose-based hydrogels prepared by freeze-thaw treatment. *Cellulose,* 2014, vol. 21 (3), 1709-1721
- Characterising the Anisotropic Mechanical Properties of Excised Human. **AISLING NÍ ANNAIDH et al.** Journal of the Mechanical Behavior of Biomedical Materials. 2012, vol. 5, 139-148
- **GALLAGHER AJ et al.** *Dynamic Tensile Properties of Human Skin, http://researchrepository.ucd.ie/handle/10197/4772*
- **SARAF H. et al.** Mechanical properties of soft human tissue under dynamic loading. *Journal of Biomechanics,* 2007, vol. 40, 1960-1967
- **TRIONA MCCORMACK ; JOSEPH M. MANSOUR.** Reduction in tensile strength of cartilage precedes surface damage under repeated compressive loading in vitro. *Journal of Biomechanics,* 1998, vol. 31, 55-61